# EUROPEAN PATENT SPECIFICATION

(11) **EP 3 689 312 B1**
(45) Date of publication and mention of the grant of the patent: **17.08.2022**
(21) Application number: 18860455.7
(22) Date of filing: 31.08.2018
(51) Int. Cl.: A61F 13/514, A61L 15/28

(54) **ABSORBENT ARTICLE**
ABSORBIERENDER ARTIKEL
ARTICLE ABSORBANT

(30) Priority: 27.09.2017 JP 2017187214
(43) Date of publication of application: 05.08.2020
(73) Proprietor: Daio Paper Corporation, Shikokuchuo-shi, Ehime 799-0492 (JP)
(72) Inventor: FURUKAWA, Masashi, Shikokuchuo-shi Ehime 799-0431 (JP); ONO, Yohei, Shikokuchuo-shi Ehime 799-0431 (JP)
(74) Representative: Held, Stephan
(86) International application number: PCT/JP2018/032381
(87) International publication number: WO 2019/065082

(56) References cited:
- JP-A- S5 545 876
- JP-A- 2002 000 657
- JP-A- 2007 504 373
- JP-A- 2007 521 841
- JP-A- 2015 120 338
- JP-A- 2016 019 566
- JP-A- 2016 222 878

## Description

### Technical Field

The present invention relates to an absorbent article due to which a product outer surface or an underwear does not easily impart a moist feel.

### Background Art

An absorbent article such as a general disposable diaper or sanitary napkin including an absorber for absorbing an excrement liquid such as urine and a liquid impervious resin film covering a back surface side of the absorber has been used. The liquid impervious resin film has moisture perviousness in a thickness direction as the liquid impervious resin film in order to prevent stuffiness at the time of wearing (see, for example, Patent Literatures 1 and 2). In many absorbent articles, in order to impart a cloth-like texture and a cloth-like appearance to a product outer surface, a back surface side of a liquid impervious resin film has been covered with an exterior nonwoven fabric (see, for example, Patent Literature 2).

However, in a conventional absorbent article, due to high moisture perviousness of a liquid impervious resin film for prevention of stuffiness, for example, after an absorber absorbs an excrement liquid, a product outer surface or an underwear retains moisture, and a user may feel feel erroneously that leakage occurs when touching the product outer surface or the underwear with his or her hand.

This problem is solved by reducing moisture perviousness of the liquid impervious resin film. However, in this case, reduction in a stuffiness-preventing property cannot be avoided.

### Citation List

### Patent Literature

Patent Literature 1: JP 2017-080028 A
Patent Literature 2: JP 2017-144174 A

### Summary of Invention

### Technical Problem

Therefore, a main object of the present invention is to provide an absorbent article due to which a product outer surface or an underwear does not easily impart a moist feel while suppressing reduction in a stuffiness-preventing property.

### Solution to Problem

Various aspects of an absorbent article that has solved the above problem are as follows.

### <First aspect>

An absorbent article including: an absorber; a liquid impervious resin film covering a back surface side of the absorber; and an exterior nonwoven fabric covering a back surface side of the liquid impervious resin film,
the liquid impervious resin film having moisture perviousness in a thickness direction, in which
a cellulose nanofiber layer is disposed between the liquid impervious resin film and the exterior nonwoven fabric.

### (Action and effect)

The cellulose nanofiber layer is obtained by applying a cellulose nanofiber aqueous solution to a target surface and then drying the cellulose nanofiber aqueous solution, and has moisture perviousness. The cellulose nanofiber has many OH groups on a surface thereof and has high hygroscopicity.

Therefore, when the cellulose nanofiber layer is disposed between the liquid impervious resin film and the exterior nonwoven fabric, moisture that has passed through the liquid impervious resin film is not blocked by the cellulose nanofiber layer. Therefore, a stuffiness-preventing property is not easily reduced at the time of wearing. Furthermore, due to high hygroscopicity of the cellulose nanofiber, more moisture is retained in the cellulose nanofiber layer than in an exterior nonwoven fabric forming a product outer surface or an underwear. Therefore, the product outer surface or the underwear does not easily impart a moist feel.

### <Second Aspect>

The absorbent article according to the first aspect, in which the cellulose nanofiber layer is formed on an outer surface of the liquid impervious resin film.

### (Action and effect)

The liquid impervious resin film having moisture perviousness in a thickness direction does not have hygroscopicity. Therefore, the exterior nonwoven fabric easily imparts a moist feel. Therefore, the cellulose nanofiber layer can also be formed on an inner surface of the exterior nonwoven fabric. However, if the cellulose nanofiber layer is formed on an outer surface of the liquid impervious resin film, the exterior nonwoven fabric is less likely to impart a moist feel. In addition, when the cellulose nanofiber layer is formed on the moisture pervious resin film, the cellulose nanofiber layer is formed into a film. Therefore, a water blocking property of the liquid impervious resin film and strength thereof are improved, and elongation is reduced advantageously.

### <Third Aspect>

The absorbent article according to the second aspect, in which the liquid impervious resin film is a moisture pervious resin film of 10 to 25 g/m².

### (Action and effect)

When the cellulose nanofiber layer is formed on the moisture pervious resin film, the water blocking property of the moisture pervious resin film and the strength thereof are improved, but the moisture pervious resin film is hard. Therefore, it is desirable that the basis weight of a base material, that is, the basis weight of the moisture pervious resin film is reduced to compensate for reduction in softness. Usually, reduction in the basis weight of the moisture pervious resin film to such a level may increase a possibility of generation of pinholes and may reduce a water blocking property. However, formation of the cellulose nanofiber layer on the moisture pervious resin film can prevent such a reduction in water blocking property.

### <Fourth Aspect>

The absorbent article according to any one of the first to third aspects, in which the cellulose nanofiber layer is formed only in an intermediate region when the absorbent article is divided equally into three portions of a front region, the intermediate region, and a back region in a front-back direction in an unfolded state.

### (Action and effect)

The cellulose nanofiber layer is hard. Therefore, when the cellulose nanofiber layer is disposed in an excessively broad range, softness of a product may be impaired. Therefore, as in the present aspect, preferably, the cellulose nanofiber layer is disposed only in a region where a product outer surface or an underwear easily imparts a moist feel, that is, only in an intermediate region when the absorbent article is divided equally into three portions of a front region, the intermediate region, and a back region in a front-back direction in an unfolded state to suppress reduction in softness in the other regions.

### <Fifth Aspect>

The absorbent article according to any one of the first to fourth aspects, in which the cellulose nanofiber layers are disposed at many places at intervals.

### (Action and effect)

The cellulose nanofiber layer is hard. Therefore, when the cellulose nanofiber layer is disposed in an excessively broad range, softness of a product may be impaired. Therefore, as in the present aspect, many cellulose nanofiber layers are preferably disposed at many places at intervals to suppress reduction in softness.

### <Sixth Aspect>

The absorbent article according to any one of the first to fifth aspects, in which the cellulose nanofiber layer contains cellulose nanofibers of 0.1 to 5.0 g/m².

### (Action and effect)

In order to improve hygroscopicity, use of a larger amount of cellulose nanofibers is more preferable. However, when the amount is too large, a product is unnecessarily hard. The content of cellulose nanofibers in the cellulose nanofiber layer is preferably within the above range.

### <Seventh Aspect>

The absorbent article according to any one of the first to sixth aspects, in which the exterior nonwoven fabric is a water repellent nonwoven fabric.

### (Action and effect)

The exterior nonwoven fabric is preferably a water repellent nonwoven fabric because hygroscopicity due to the cellulose nanofiber layer is further increased. Advantageous Effects of Invention

The present invention advantageously provides an absorbent article due to which a product outer surface or an underwear does not easily impart a moist feel while suppressing reduction in a stuffiness-preventing property.

### Brief Description of Drawings

Fig. 1 is a plan view illustrating an inner surface of a tape-type disposable diaper in a state where the diaper is unfolded.
Fig. 2 is a plan view illustrating an outer surface of a tape-type disposable diaper in a state where the diaper is unfolded.
Fig. 3 is a cross-sectional view cut along 6-6 of Fig. 1.
Fig. 4 is a cross-sectional view cut along 7-7 of Fig. 1.
Fig. 5 is a cross-sectional view cut along 8-8 of Fig. 1.
Fig. 6 is a cross-sectional view cut along 9-9 of Fig. 1.
Fig. 7 is a cross-sectional view cut along 5-5 of Fig. 1.
Fig. 8 is a cross-sectional view illustrating a main part.
Fig. 9 is a cross-sectional view illustrating a main part.
Fig. 10 is a cross-sectional view illustrating a main part.
Fig. 11 is a plan view illustrating an outer surface of a tape-type disposable diaper in a state where the diaper is unfolded.
Fig. 12 is a plan view illustrating arrangement of cellulose nanofiber layers.

### Description of Embodiments

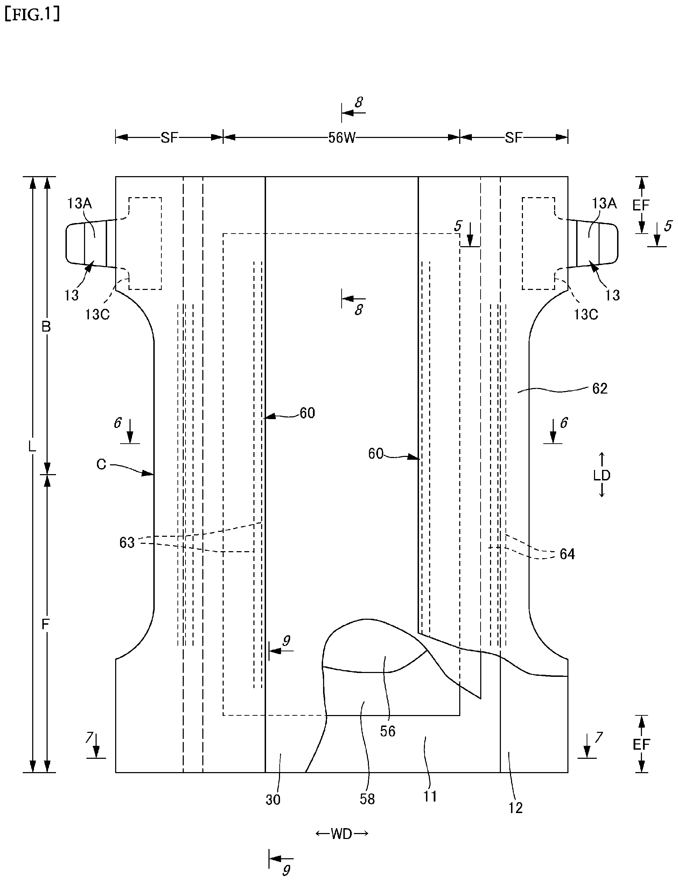

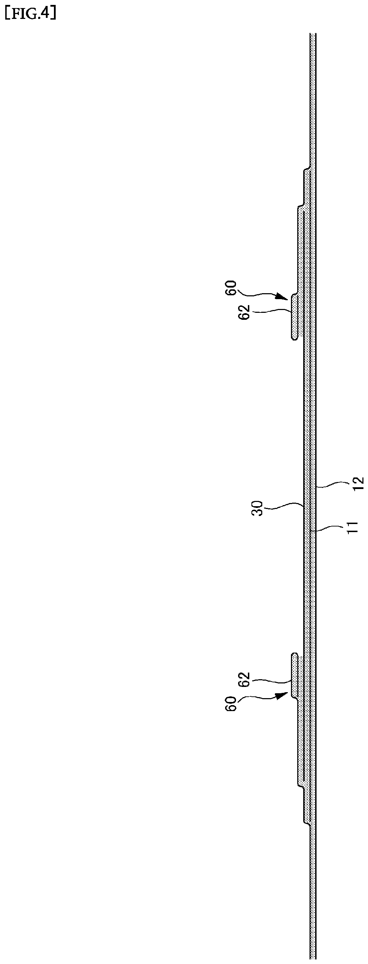

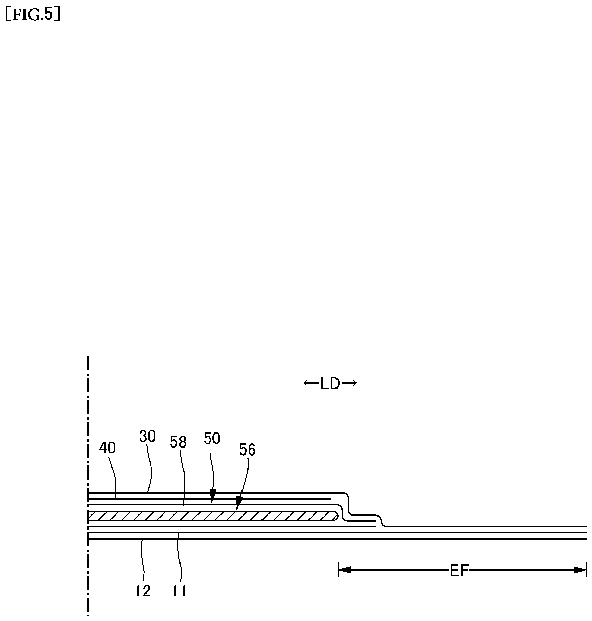

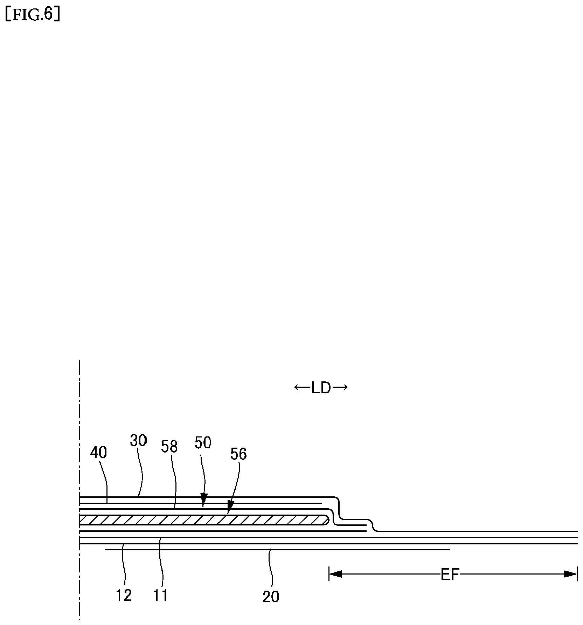

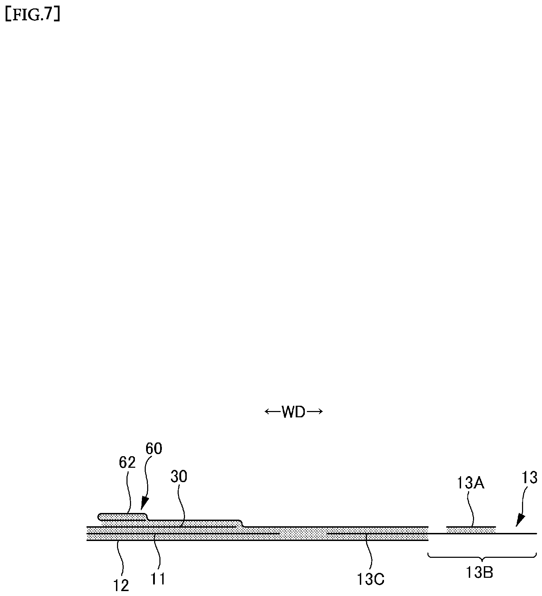

Figs. 1 to 7 illustrate an example of a tape-type disposable diaper. A reference character X in the drawings represents the maximum width of the diaper excluding a connecting tape. A reference character L represents the maximum length of the diaper. A dotted pattern portion in the cross-sectional views illustrates an adhesive as a bonding means for bonding constituent members located on a front surface side and a back surface side. The constituent members are formed by applying a hot melt adhesive by solid application, bead application, curtain application, summit application, spiral application, pattern coating (transfer of a hot melt adhesive by a letterpress method), or the like. Alternatively, a fixing portion of an elastic member is formed, instead of this or in addition to this, by application to an outer peripheral surface of an elastic member by a comb gun, SureWrap application, or the like. Examples of the hot melt adhesive include an EVA-based agent, a pressure-sensitive rubber-based agent (elastomer-based agent), a polyolefin-based agent, and a polyester/polyamide-based agent, and these can be used without particular limitation. As a bonding means for bonding constituent members, a means by material welding such as heat sealing or ultrasonic sealing can also be used.

This tape-type disposable diaper includes an absorber 56, a liquid pervious top sheet 30 covering a front surface side of the absorber 56, a liquid impervious resin film 11 covering a back side of the absorber 56, and an exterior nonwoven fabric 12 covering a back surface side of the liquid impervious resin film and forming a product outer surface. A reference character F represents a ventral side portion located in a front side of the center in the front-back direction. A reference character B represents a dorsal side portion located in a back side of the center in the front-back direction.

Hereinafter, a material of each portion and a characteristic part thereof will be described sequentially.

### (Absorber)

The absorber 56 absorbs and holds an excrement liquid, and can be formed by an assembly of fibers. As this fiber assembly, in addition to those obtained by accumulating short fibers such as fluff pulps or synthetic fibers, a filament assembly obtained by opening a tow (fiber bundle) of synthetic fibers such as cellulose acetate as necessary can also be used. In a case where fluff pulps or short fibers are accumulated, a fiber basis weight may be, for example, about 100 to 300 g/m². In a case of a filament assembly, a fiber basis weight may be, for example, about 30 to 120 g/m². In a case of a synthetic fiber, a fineness is, for example, 1 to 16 dtex, preferably 1 to 10 dtex, and more preferably 1 to 5 dtex. In a case of a filament assembly, the filament may be formed of non-crimped fibers but is preferably formed of crimped fibers. The degree of crimp of the crimped fibers may be, for example, about 5 to 75, preferably 10 to 50, and more preferably 15 to 50 per 2.54 cm. In addition, a uniformly crimped fiber can be used.

### (Super absorbent polymer particles)

The absorber 56 may contain super absorbent polymer particles partially or entirely. The super absorbent polymer particles include "powder" in addition to "particles". As super absorbent polymer particles 54, those used for this type of absorbent article can be used as they are. The particle diameters of the super absorbent polymer particles are not particularly limited. However, for example, when sieving using a standard sieve of 500 µm (JIS Z8801-1: 2006) (shake for five minutes) is performed, and particles falling under the sieve using this sieving are sieved using a standard sieve of 180 µm (JIS Z8801-1: 2006) (shake for five minutes), it is desirable that a ratio of particles remaining on the standard sieve of 500 µm is 30% by weight or less, and a ratio of particles remaining on the standard sieve of 180 µm is 60% by weight or more.

A material of the super absorbent polymer particles can be used without particular limitation, but those having a water absorption capacity of 30 g/g or more are preferable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

As the super absorbent polymer particles, those having a water absorption rate of 70 seconds or less, particularly 40 seconds or less are suitably used. When the water absorption rate is too slow, so-called returning that a liquid supplied into the absorber 56 returns out of the absorber 56 tends to occur.

As the super absorbent polymer particles, those having a gel strength of 1000 Pa or more are suitably used. This makes it possible to effectively suppress sticky feeling after liquid absorption even in a case of using the bulky absorber 56.

The basis weight of the super absorbent polymer particles can be appropriately determined depending on the absorption amount required for an application of the absorber 56. Therefore, the basis weight can be 50 to 350 g/m² although this cannot be applied generally. The basis weight of a polymer of less than 50 g/m² makes it difficult to secure the absorption amount. When the basis weight exceeds 350 g/m², not only the effect is saturated but also the excess of the super absorbent polymer particles imparts a gritty and uncomfortable feel.

### (Wrapping sheet)

The absorber 56 can be incorporated as an absorbent element 50 wrapped in a wrapping sheet 58 in order to prevent escape of the super absorbent polymer particles or to improve shape maintenance of the absorber 56. As the wrapping sheet 58, tissue paper, particularly crepe paper, a nonwoven fabric, a polylaminated nonwoven fabric, a sheet with small holes, and the like can be used. However, it is desirable that the wrapping sheet 58 is a sheet from which super absorbent polymer particles do not escape. When a nonwoven fabric is used instead of crepe paper, a hydrophilic spunbonded/melt blown/melt blown/spunbonded (SMMS) nonwoven fabric is particularly suitable, and polypropylene, polyethylene/polypropylene, or the like can be used as a material thereof. A nonwoven fabric having a fiber basis weight of 5 to 40 g/m², particularly of 10 to 30 g/m² is desirable.

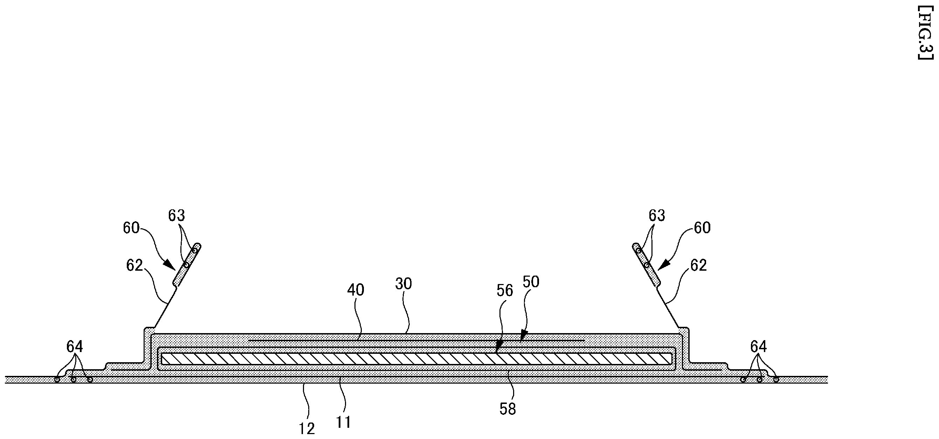

As illustrated in Fig. 3, the single wrapping sheet 58 may wrap the whole of the absorber 56, or a plurality of the wrapping sheets 58 such as upper and lower two wrapping sheets 58 may wrap the whole of the absorber 56. The wrapping sheet can be omitted.

### (Top sheet)

As the top sheet 30, a liquid pervious sheet, for example, a perforated or imperforated nonwoven fabric or a porous plastic sheet can be used. Among these materials, the nonwoven fabric is not particularly limited concerning a raw material fiber thereof. Examples thereof include a synthetic fiber such as a polyolefin-based fiber including polyethylene and polypropylene, a polyester-based fiber, or a polyamide-based fiber, a regenerated fiber such as rayon or cupra, a natural fiber such as cotton, and a mixed fiber and a composite fiber in which two or more kinds of these fibers are used. Furthermore, the nonwoven fabric may be manufactured by any processing. Examples of a processing method include known methods such as a spunlacing method, a spunbonding method, a thermal bond method, a melt blown method, a needle punching method, an air through method, and a point bond method. For example, if softness and drapeability are demanded, a spunlacing method is a preferable processing method. If bulkiness and softness are demanded, a thermal bond method is a preferable processing method.

The top sheet 30 extends from a front end to a back end of the product in the front-back direction and extends to a lateral side more than the absorber 56 in the width direction WD. For example, when a starting point of a rising gather 60 described later is located closer to the center in the width direction than a side edge of the absorber 56, appropriate deformation can be made, for example, the width of the top sheet 30 is made shorter than the maximum width of the absorber 56 as necessary.

### (Intermediate sheet)

In order to quickly transfer a liquid that has passed through the top sheet 30 to the absorber, it is possible to dispose the intermediate sheet (also referred to as "second sheet") 40 having a higher liquid transmission rate than the top sheet 30. The intermediate sheet 40 is used in order to rapidly transfer a liquid to the absorber to enhance absorption performance of the absorber, and to prevent a "returning" phenomenon of the absorbed liquid from the absorber. The intermediate sheet 40 can be omitted.

Examples of the intermediate sheet 40 include a similar material to that of the top sheet 30, a spunlaced nonwoven fabric, a spunbonded nonwoven fabric, an SMS nonwoven fabric, a pulp nonwoven fabric, a mixed sheet of pulp and rayon, a point bonded nonwoven fabric, and crepe paper. In particular, an air through nonwoven fabric is preferable because of being bulky. As the air through nonwoven fabric, a composite fiber having a core-sheath structure is preferably used. In this case, a resin used for the core may be polypropylene (PP) but is preferably polyester (PET) having high rigidity. The basis weight is preferably 17 to 80 g/m², and more preferably 25 to 60 g/m². A raw material fiber of the nonwoven fabric preferably has a fineness of 2.0 to 10 dtex. In order to make the nonwoven fabric bulky, as mixed fibers of all or some of raw material fibers, eccentric fibers having no core in the center, hollow fibers, eccentric and hollow fibers are also preferably used.

The intermediate sheet 40 in the illustrated example is disposed at the center so as to be shorter than the width of the absorber 56, but may be disposed over the maximum width. The intermediate sheet 40 may be disposed over the maximum length of the diaper, but may be disposed only in an intermediate portion including an excrement position as in the illustrated example.

### (Liquid impervious resin film)

The liquid impervious resin film 11 is not particularly limited as long as having moisture perviousness. However, for example, a microporous sheet obtained by kneading an inorganic filler in a polyolefin-based resin such as polyethylene or polypropylene, molding into a sheet, and then stretching the sheet in a monoaxial or biaxial direction can be used suitably. Needless to say, the liquid impervious resin film 11 does not include a liquid impervious resin film including a nonwoven fabric as a base material to enhance waterproofness.

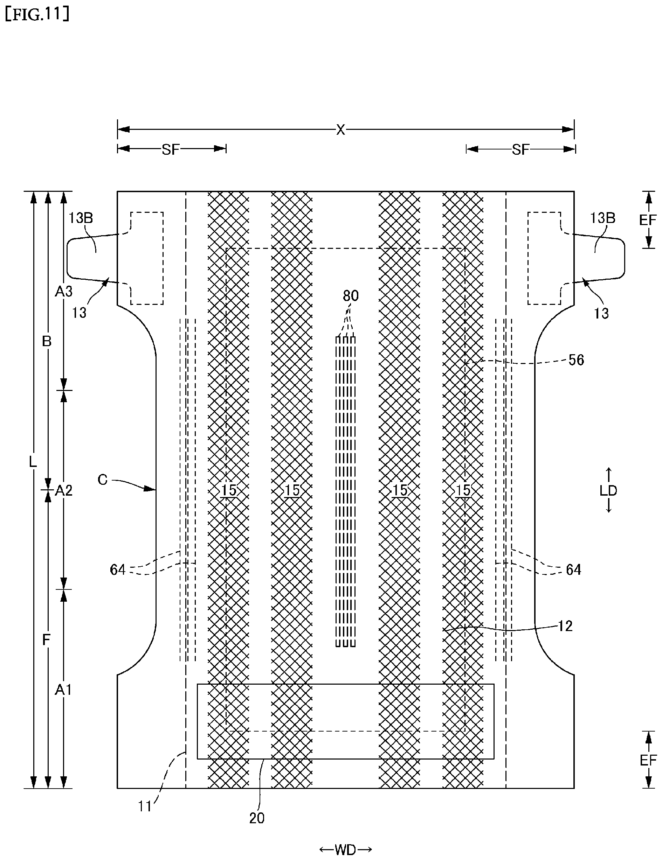

It is desirable that the liquid impervious resin film 11 extends within the same range as or a wider range than the absorber 56 in the front-back direction LD and the width direction WD. However, for example, when another water blocking means is present, an end portion of the absorber 56 does not have to be covered in the front-back direction LD and the width direction WD as necessary. On a surface of the liquid impervious resin film 11, as illustrated in Fig. 11, an indicator 80 that is colored or decolored by a liquid component of excrement can be disposed. As the indicator 80, a known indicator can be used without particular limitation. For example, the indicator 80 can be formed by a sheet-like member containing a coloring agent that exhibits a color reaction by contact with water in excrement and/or a coloring agent that detects the pH in water to exhibit a color reaction, an ink or an adhesive containing a chemical agent that exhibits a discoloration reaction due to a reaction with a liquid in excrement, a reaction that causes spread or discoloration of a coloring agent due to dissolution (dispersion) of the coloring agent by urine, or another visual change, or a chemical agent that exhibits a visual change by contact with water or a liquid in excrement (indicator reaction means). As the coloring agent that exhibits a color reaction by contact with water in excrement, a coloring agent containing a water-soluble or water-degradable dye or a leuco dye and a developer such as a phenolic compound, an acid substance, or an electron-accepting substance that develops a color of the leuco dye can be used.

### (Exterior nonwoven fabric)

The exterior nonwoven fabric 12 covers the entire back surface side of the liquid impervious resin film 11 and imparts a cloth-like appearance to a product outer surface. The exterior nonwoven fabric 12 is not particularly limited. Examples thereof as a material fiber include a synthetic fiber such as a polyolefin-based fiber including polyethylene and polypropylene, a polyester-based fiber, or a polyamide-based fiber, a regenerated fiber such as rayon or cupra, and a natural fiber such as cotton. Examples of a processing method include a spunlacing method, a spunbonding method, a thermal bond method, an air through method, and a needle punching method. However, a long-fiber nonwoven fabric such as a spunbonded nonwoven fabric, an SMS nonwoven fabric, or an SMMS nonwoven fabric is suitable from a viewpoint of achieving both texture and strength. A nonwoven fabric can be used in a single sheet or in a plurality of laminated sheets. In the latter case, the nonwoven fabrics are preferably bonded to each other with a hot melt adhesive or the like. In a case where a nonwoven fabric is used, it is desirable that the nonwoven fabric has a fiber basis weight of 10 to 50 g/m², particularly 15 to 30 g/m².

### (Rising gather)

In order to block excrement that moves laterally on the top sheet 30 and to prevent so-called side leakage, rising gathers 60 rising on a skin side of a wearer are preferably disposed on both sides of a surface in the width direction WD. Of course, the rising gathers 60 can be omitted.

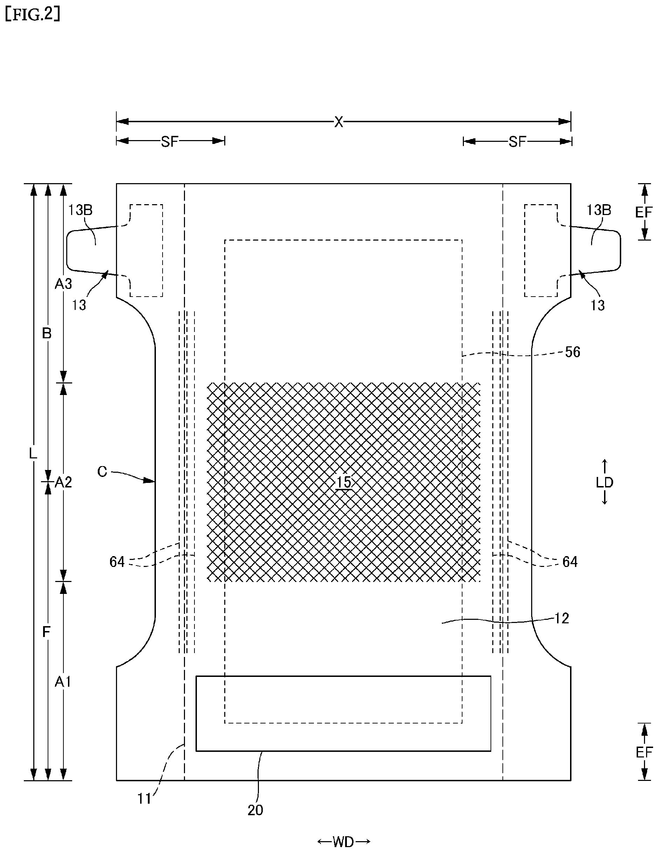

When the rising gather 60 is adopted, a structure thereof is not particularly limited, and any known structure can be adopted. The rising gather 60 in the illustrated example includes a gather sheet 62 substantially continuous in the width direction WD, and an elongated gather elastic member 63 fixed to the gather sheet 62 in a stretched state in the front-back direction LD. A water repellent nonwoven fabric can be used as the gather sheet 62, and a rubber thread or the like can be used as the gather elastic member 63. As illustrated in Figs. 1 and 2, a plurality of elastic members may be disposed, or one elastic member may be disposed.

An inner surface of the gather sheet 62 has a bonding start point in the width direction WD on a side portion of the top sheet 30. A portion from the bonding start point to the outside in the width direction is bonded to an inner surface of each side flap portion SF, that is, in the illustrated example, bonded to a side portion of the liquid impervious resin film 11 and a side portion of the exterior nonwoven fabric 12 located in an outside thereof in the width direction with a hot melt adhesive or the like.

In a periphery of a leg, an inside of the bonding start point of the rising gather 60 in the width direction is fixed to the top sheet 30 at both end portions in a product front-back direction. However, a portion therebetween is a non-fixed free portion, and the free portion rises by a contraction force of the elastic member 63 and comes into close contact with a body surface.

### (End flap portion and side flap portion)

The tape-type disposable diaper in the illustrated example has a pair of end flap portions EF not including the absorber 56 and extending to a front side and a back side of the absorber 56, and a pair of side flap portions SF not including the absorber 56 and extending to a lateral side more than both side edges of the absorber 56.

### (Plane gather)

To each side flap portion SF, a side elastic member 64 formed of an elongated elastic member such as a rubber thread is fixed in a stretched state in the front-back direction LD, and a periphery of a leg of each side flap portion SF is configured as a plane gather. The leg periphery elastic member 64 can be disposed between the gather sheet 62 and the liquid impervious resin film 11 outside the vicinity of the bonding start point in the width direction in the bonded portion of the gather sheet 62 as in the illustrated example, and can also be disposed between the liquid impervious resin film 11 and the exterior nonwoven fabric 12 in the side flap portion SF. A plurality of the leg periphery elastic members 64 may be disposed on each side as in the illustrated example, or only one leg periphery elastic member 64 may be disposed on each side.

### (Connecting tape)

In each side flap portion SF of the dorsal side portion B, a connecting tape 13 to be detachably connected to an outer surface of the ventral side portion F is disposed. When the diaper 10 is worn, the connecting tapes 13 are taken from both sides of a waist to an outer surface of the ventral side portion F, and the connecting portions 13A of the connecting tapes 13 are connected to appropriate positions of the outer surface of the ventral side portion F.

The structure of the connecting tape 13 is not particularly limited. However, in the illustrated example, the connecting tape 13 includes: a tape attachment portion 13C fixed to the side flap portion SF; a sheet base material forming a tape main unit portion 13B protruding from the tape attachment portion 13C; and the ventral side connecting portion 13A disposed in a width direction intermediate portion of the tape main unit portion 13B in the sheet base material, and a tip side of the connecting portion 13A is a tab part.

As the connecting portion 13A, a hook material (hook member) of a mechanical fastener (hook and loop fastener) or a pressure-sensitive adhesive layer may be disposed. The hook material has many engaging projections on a connecting surface thereof. Examples of the shapes of the engaging projections include (A) tick shape, (B) J shape, (C) mushroom shape, (D) T shape, and (E) double J shape (a shape in which the J-shaped ones are connected to each other back to back), but any shape may be used.

As the sheet base material forming from the tape attachment portion 13C to the tape main unit portion 13B, a nonwoven fabric, a plastic film, a polylaminated nonwoven fabric, paper, or a composite material thereof can be used. However, a spunbonded nonwoven fabric, an air through nonwoven fabric, or a spunlaced nonwoven fabric having a fineness of 1.0 to 3.5 dtex, a basis weight of 20 to 100 g/m², and a thickness of 1 mm or less is preferable.

### (Target sheet)

A target sheet 20 having a target for facilitating connection is preferably disposed at a connecting location of the connecting tape 13 in the ventral side portion F. In a case where the connecting portion 13A is formed of a hook material, as the target sheet 20, it is possible to use one in which many loop threads making engaging projections of the hook material entangled therewith are disposed on a surface of a sheet base material formed of a plastic film or a nonwoven fabric. In a case of a pressure-sensitive material layer, it is possible to use one obtained by subjecting a surface of a sheet base material formed of a plastic film having a smooth surface with high pressure-sensitive adhesiveness to a peeling treatment. When the connecting location of the connecting tape 13 in the ventral side portion F is formed of a nonwoven fabric, for example, when the exterior nonwoven fabric 12 is disposed as in the illustrated embodiment, the target sheet 20 can be omitted, and the hook material can be entangled with fibers of the exterior nonwoven fabric 12 to be connected. In this case, the target sheet 20 as a mark may be disposed between the exterior nonwoven fabric 12 and the liquid impervious resin film 11.

### (Cellulose nanofiber layer)

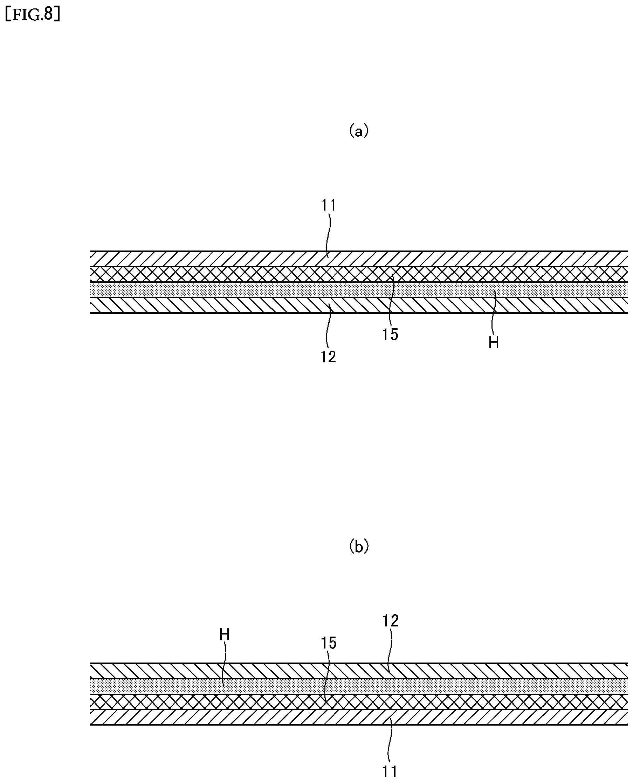

Characteristically, as illustrated in Figs. 8 to 10, a cellulose nanofiber layer 15 is disposed between the liquid impervious resin film 11 and the exterior nonwoven fabric 12. Note that a reference character H in Figs. 8 to 10 represents an adhesive such as a hot melt adhesive for bonding the liquid impervious resin film 11 to the exterior nonwoven fabric 12. However, as described above, the liquid impervious resin film 11 may be bonded to the exterior nonwoven fabric 12 by welding or the like of a material. When such a cellulose nanofiber layer 15 is disposed between the liquid impervious resin film 11 and the exterior nonwoven fabric 12, moisture that has passed through the liquid impervious resin film 11 is not blocked by the cellulose nanofiber layer 15. Therefore, a stuffiness-preventing property is not easily reduced at the time of wearing. Furthermore, due to high hygroscopicity of the cellulose nanofiber, more moisture is retained in the cellulose nanofiber layer 15 than in the exterior nonwoven fabric 12 forming a product outer surface or an underwear. Therefore, the product outer surface or the underwear does not easily impart a moist feel. In addition, the cellulose nanofiber layer 15 has no or low air permeability and has an effect of blocking odor of excrement, and some odor components are adsorbed by the cellulose nanofiber layer 15. As a result, release of odor of excrement is also reduced.

In this case, the exterior nonwoven fabric 12 is preferably a water repellent nonwoven fabric because hygroscopicity due to the cellulose nanofiber layer 15 is further increased. The water repellent nonwoven fabric can be manufactured by adding a water repellent agent to a nonwoven fabric by a known method (either internal addition or external addition may be used). Examples of the water repellent agent include a silicone-based water repellent agent, a paraffin-based water repellent agent, and an alkylchromic chloride-based water repellent agent.

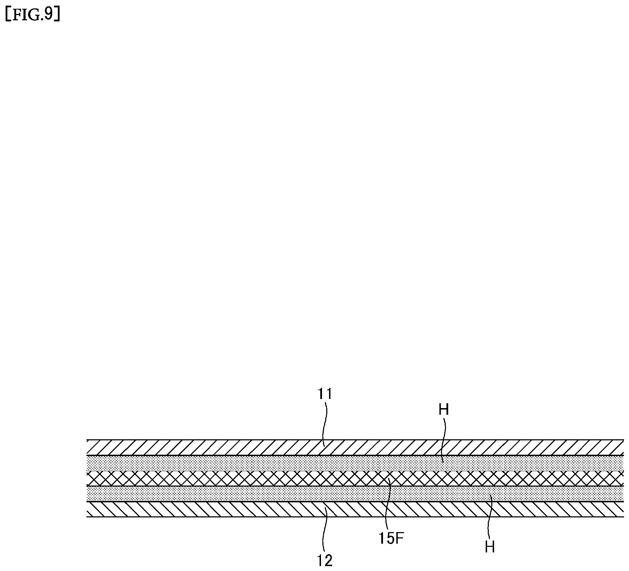

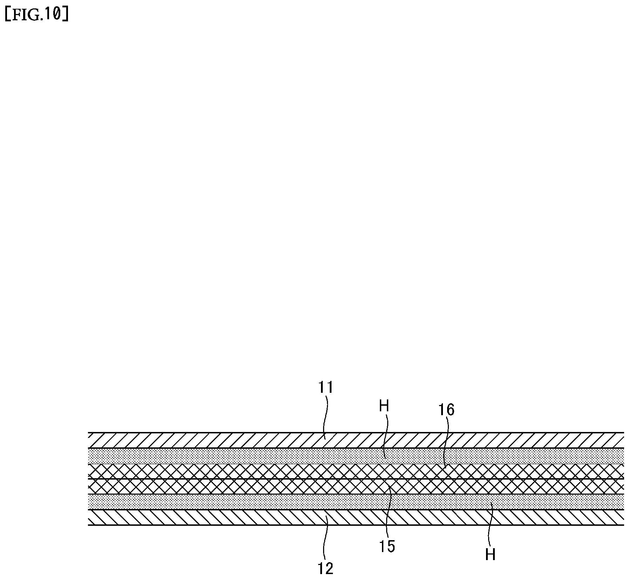

As illustrated in Fig. 8(b), the cellulose nanofiber layer 15 can be formed on an inner surface of the exterior nonwoven fabric 12. As illustrated in Fig. 9, a cellulose nanofiber film 15F can be disposed between the liquid impervious resin film 11 and the exterior nonwoven fabric 12. As illustrated in Fig. 10, a sheet 16 of a nonwoven fabric, paper, or the like having the cellulose nanofiber layer 15 formed thereon can be disposed between the liquid impervious resin film 11 and the exterior nonwoven fabric 12. However, as illustrated in Fig. 8(a), the cellulose nanofiber layer 15 is preferably formed on an outer surface of the liquid impervious resin film 11. In particular, since the liquid impervious resin film 11 does not have hygroscopicity, the exterior nonwoven fabric 12 easily imparts a moist feel. However, when the cellulose nanofiber layer 15 is disposed on an outer surface of the liquid impervious resin film 11, the exterior nonwoven fabric 12 does not easily impart a moist feel. In addition, when the cellulose nanofiber layer 15 is formed on the moisture pervious resin film 11 formed of a moisture pervious resin film, the cellulose nanofiber layer 15 is formed into a film. Therefore, a water blocking property of the liquid impervious resin film 11 and strength thereof are improved, and elongation is reduced advantageously. In particular, on the liquid impervious resin film 11 formed of a moisture pervious resin film, continuous decorative printing including many constituent units such as letters (size, brand name, manufacturer's name, pattern name, and the like) regularly repeated in the front-back direction LD and the width direction WD, or intermittent decorative printing for printing only one or both of the front and back of a product as in a product logo, a picture of a character, or a photograph may be performed. However, when such decorative printing is performed, the elongation of the liquid impervious resin film 11 is desirably small.

When the cellulose nanofiber layer 15 is formed on the liquid impervious resin film 11 formed of a moisture pervious resin film, the water blocking property of the liquid impervious resin film 11 and the strength thereof are improved, but the liquid impervious resin film 11 is hard. Therefore, it is desirable that the basis weight of a base material of the liquid impervious resin film 11, that is, the basis weight of the moisture pervious resin film is reduced to, for example, about 10 to 12 g/m² to compensate for reduction in softness. Usually, reduction in the basis weight of the moisture pervious resin film to such a level may increase a possibility of generation of pinholes and may reduce a water blocking property. However, formation of the cellulose nanofiber layer 15 on the moisture pervious resin film can prevent such a reduction in water blocking property.

It is desirable that the cellulose nanofiber layer 15 extends in the same range as or a wider range than the absorber 56 or the absorbing element 50 in the front-back direction LD and the width direction WD. However, the cellulose nanofiber layer 15 may be disposed only in a narrower range than the absorber 56 or the absorbent element 50 in at least one of the front-back direction LD and the width direction WD as necessary. The cellulose nanofiber layer 15 may extend in the entire liquid impervious resin film 11 or in a wider range than the entire liquid impervious resin film 11 in the front-back direction LD and the width direction WD. However, the cellulose nanofiber layer 15 is hard. Therefore, the cellulose nanofiber layer 15 is also preferably disposed only in a partial region in at least one of the front-back direction LD and the width direction WD. For example, as illustrated in Fig. 2, preferably, the cellulose nanofiber layer 15 is disposed only in a region where a product outer surface or an underwear easily imparts a moist feel in the front-back direction LD, that is, only in an intermediate region A2 when the absorbent article is divided equally into three portions of a front region A1, the intermediate region A2, and a back region A3 in the front-back direction LD in an unfolded state to suppress reduction in softness in the other regions. Both end portions of the product in the width direction WD are in contact with a periphery of a leg. Therefore, as illustrated in Fig. 2, it is desirable that the cellulose nanofiber layer 15 is not be disposed at both end portions of the product in the width direction WD and disposed only in a region therebetween. For example, when the liquid impervious resin film 11 does not extend to both end portions of the product in the width direction WD, it is similarly desirable that the cellulose nanofiber layer 15 is disposed over the entire liquid impervious resin film 11 in the width direction WD.

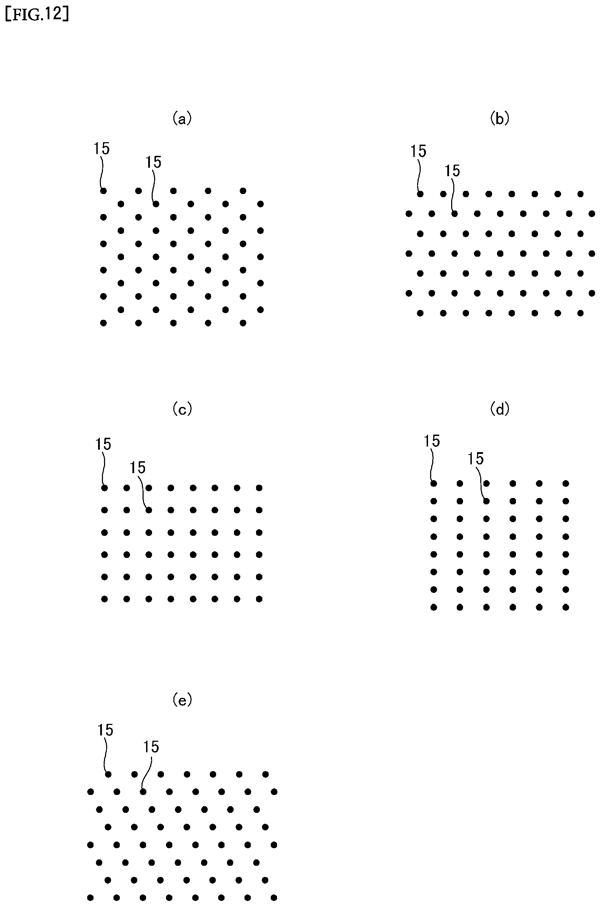

In order to increase softness without reducing hygroscopicity, the cellulose nanofiber layers 15 are preferably disposed at many places at intervals. For example, as illustrated in Fig. 11, the cellulose nanofiber layers 15 can be disposed in a form of vertical stripes. In addition, although not illustrated, the cellulose nanofiber layers 15 can be disposed in a form of horizontal stripes, in a form of diagonal stripes, or in a form of lattices. As illustrated in Fig. 12, the cellulose nanofiber layers 15 can be disposed in a form of scattering points. As the planar shape of the cellulose nanofiber layer 15 in this case, for example, in addition to a true circle and an ellipse, any shape such as a polygon including a triangle, a rectangle, and a rhombus, a star, and a cloud can be adopted. The planar arrangement of the cellulose nanofiber layers 15 can be determined appropriately. However, a regularly repeated arrangement such as an orthorhombic lattice as illustrated in Fig. 12(a), a hexagonal lattice as illustrated in Fig. 12(b) (these are also referred to as a staggered form), a square lattice as illustrated in Fig. 12(c), a rectangular lattice as illustrated in Fig. 12(d), a parallel lattice as illustrated in Fig. 14(e) (a form in which two groups each including many parallel and diagonal rows are disposed so as to cross each other as illustrated), or a form in which these are inclined at an angle of less than 90 degrees with respect to the front-back direction is preferable. However, the arrangement may be irregular.

In order to improve hygroscopicity, use of a larger amount of cellulose nanofibers is more preferable. However, when the amount is too large, a product is unnecessarily hard. Therefore, the content of cellulose nanofibers in the cellulose nanofiber layer 15 is preferably about 0.1 to 5.0 g/m².

The cellulose nanofiber layer 15 can be manufactured by a known method such as a method for forming cellulose nanofibers into a cellulose nanofiber aqueous solution, applying this cellulose nanofiber aqueous solution onto a target sheet such as the liquid impervious resin film 11, and drying the cellulose nanofiber aqueous solution to attach and form the cellulose nanofibers onto the target sheet. Note that when a liquid cellulose nanofiber is applied to a fiber sheet such as paper or a nonwoven fabric by a manufacturing method for applying a liquid cellulose nanofiber in this way, a part of the liquid cellulose nanofiber penetrates the sheet, but cellulose nanofibers concentrate on a surface. Therefore, the cellulose nanofiber layer 15 can be attached and formed onto the sheet.

The cellulose nanofiber aqueous solution is obtained by dispersing cellulose nanofibers in water. The concentration (mass/volume) of the cellulose nanofiber aqueous solution is preferably 0.1 to 10%, more preferably 1.0 to 5.0%, and particularly preferably 1.5 to 3.0%.

The B-type viscosity (60 rpm, 20°C) of the cellulose nanofiber aqueous solution is, for example, 300 cps or less, preferably 200 cps or less, and more preferably 50 cps or less. By suppressing the B-type viscosity of the cellulose nanofiber aqueous solution to a low level in this way, the cellulose nanofibers can be uniformly applied to a sheet surface, and the surface properties of the sheet are uniformly improved.

The cellulose nanofibers can be applied not only by spraying the cellulose nanofibers onto a target surface but also by using a transfer method with a letterpress method or the like.

The cellulose nanofibers refer to fine cellulose fibers obtained by defibrating pulp fibers, and generally refer to cellulose fibers containing cellulose fine fibers having an average fiber width of nano size (1 nm or more and 1000 nm or less). Cellulose nanofibers having an average fiber width (median diameter) of 100 nm or less are preferable, and cellulose nanofibers having 10 to 60 nm are particularly preferable.

Here, a method for measuring the average fiber width of cellulose nanofibers is described.

First, 100 ml of an aqueous dispersion of cellulose nanofibers having a solid concentration of 0.01 to 0.1% by mass is filtered through a Teflon (registered trademark) membrane filter, and solvent substitution is performed once with 100 ml of ethanol and three times with 20 ml of t-butanol.

Next, the resulting product is lyophilized and coated with osmium to obtain a sample. This sample is observed with an electron microscope SEM image at a magnification of 5000, 10000, or 30000 (in this example, a magnification of 30000) depending on the width of a fiber forming the sample. Specifically, two diagonals are drawn on the observation image, and three straight lines passing the intersection of the diagonals are arbitrarily drawn. Furthermore, a total of 100 fiber rods intersecting the three straight lines are visually measured. Then, the median diameter of the measured values is taken as an average fiber width.

Examples of pulp fibers that can be used for manufacturing cellulose nanofibers include a chemical pulp such as a leaf bleached kraft pulp (LBKP) or a needle bleached kraft pulp (NBKP), a mechanical pulp such as a bleached thermomechanical pulp (BTMP), stone ground pulp (SGP), a pressed stone ground pulp (PGW), a refiner ground pulp (RGP), a chemiground pulp (CGP), a thermoground pulp (TGP), a ground pulp (GP), a thermomechanical pulp (TMP), a chemithermomechanical pulp (CTMP), or a refiner mechanical pulp (RMP), a waste paper pulp manufactured from brown waste paper, kraft envelope paper, magazine waste paper, newspaper waste paper, flyer waste paper, office waste paper, cardboard waste paper, high-quality white waste paper, Kent waste paper, simili waste paper, landowner certificate waste paper, or woody waste paper, and deinked pulp (DIP) obtained by deinking a waste paper pulp. These pulp fibers may be used singly or in combination of two or more types thereof as long as the effects of the present invention are not impaired. Furthermore, the pulp fiber may be subjected to a chemical treatment such as carboxymethylation.

Examples of a method for manufacturing cellulose nanofibers include mechanical methods such as a high-pressure homogenizer method, a microfluidizer method, a grinder grinding method, a bead mill freeze grinding method, and an ultrasonic defibrating method, but are not limited to these methods. In addition, nanofiber formation is promoted by combined use of a TEMP oxidation treatment, a phosphoric acid esterification treatment, an acid treatment, and the like.

### <Effect confirmation test>

The following samples and the like were manufactured, and the following wetness, air permeability, and tensile tests were performed.

### (Sample diaper)

In the tape-type disposable diaper described above, a sample diaper in which the cellulose nanofiber layer 15 was formed, and a blank diaper in which the cellulose nanofiber layer was not formed were prepared. Note that the absorber 56 is obtained by mixing pulp fibers and super absorbent polymer particles uniformly, and contains 180 g/m² of pulp fibers and 220 g/m² of superabsorbent polymer particles.

As the super absorbent polymer particles, particles which had a water absorption capacity of 33 g/g, a water absorption rate of 35 seconds, and a gel strength of 3800 Pa, and in which a ratio of particles remaining on a standard sieve of 500 µm (JIS Z8801-1: 2006) was 18% by weight, and a ratio of particles remaining on a standard sieve of 180 µm (JIS Z8801-1: 2006) was 80% by weight when sieving using the standard sieve of 500 µm (shake for five minutes) was performed, and particles falling under the sieve using this sieving were sieved using the standard sieve of 180 µm (shake for five minutes), were used.

As the liquid impervious resin film 11, a moisture pervious polyethylene film having a basis weight of 18 g/m² and a moisture penetration of 9000 g/m²·24h (method of temperature and humidity condition B of JIS Z 0208 (under condition of temperature 40°C and humidity 90%)) was used.

As the exterior nonwoven fabric 12, an air through nonwoven fabric with a basis weight of 20 g/m² with a composite fiber (fineness: 2.0 dtex) having a core-sheath structure of polyethylene (sheath) and polyethylene terephthalate (core) was used.

### (Cellulose nanofiber layer)

An aqueous solution of cellulose nanofibers (cellulose nanofiber concentration: 2.0%) containing 100% of NBKP and having an average fiber width (median diameter) of 49 nm was applied uniformly to the entire back surface of the liquid impervious resin film 11 and dried to manufacture the liquid impervious resin film 11 having the cellulose nanofiber layer 15 of 0.40 g/m². The sample diaper was manufactured using this liquid impervious resin film 11. Similarly, a sample diaper with cellulose nanofibers containing 100% of NBKP and having an average fiber width (median diameter) of 300 nm was also manufactured. Note that those obtained by subjecting NBKP to a refiner treatment to be roughly defibrated were used as the above 300 nm cellulose nanofibers, and those obtained by then treating the 300 nm cellulose nanofibers four times using a high-pressure homogenizer to be defibrated were used as the above 49 nm cellulose nanofibers.

### (Wetness)

Each of the sample diapers was fixed on a horizontal test stand in an unfolded state. Between a crotch portion of each of the sample diapers and the test stand, 20 sheets of 100 mm square filter paper were laminated and sandwiched (such that the center of the filter paper overlaps with the center of the product in the front-back direction and the width direction). Into a crotch portion of the sample 1, 70 cc of artificial urine (urea: 2% by weight, sodium chloride: 0.8% by weight, calcium chloride dihydrate: 0.03% by weight, magnesium sulfate heptahydrate: 0.08% by weight, deionized water: 97.09% by weight) was injected. After the artificial urine disappeared from a surface, a weight having a bottom area of 100 cm² and a weight of 3 kg was placed on a surface of the injection portion and held for 15 minutes. Next, the filter paper was taken out and the weight thereof as measured. By subtracting the weight of the filter paper before artificial urine injection from the measured weight value, a change in the weight of the filter paper was determined. The measurement was performed four times, and an average value thereof was taken as wetness. Similarly, the wetness of a blank diaper was also measured.

### (Air permeability)

Air permeability of each of the sample diapers was measured by method A (Frazier method) according to JIS L 1096. Note that the sample diapers were used as they were without cutting out a sample. In the test, arrangement was performed such that the center of the injection portion would overlap with the center of a cylinder (diameter 70 mm) after 70 cc of artificial urine was injected into a crotch portion of each of the sample diapers and disappeared from the surface, and the test was performed by attaching the surface to one end of the cylinder (diameter 70 mm) of a tester. The measurement was performed three times, and an average value thereof was taken as air permeability. Similarly, the air permeability of a blank diaper was also measured.

### (Tensile test)

To the same liquid impervious resin film (moisture pervious polyethylene film) as used in the sample diapers, a 2.0% aqueous solution of cellulose nanofibers containing 100% of NBKP and having an average fiber width (median diameter) of 49 nm was applied and dried to manufacture a 50 mm wide × 250 mm long sample sheet having a cellulose nanofiber layer of 0.40 g/m² formed thereon. A blank sheet containing only a moisture pervious polyethylene film and having no cellulose nanofiber layer formed thereon was prepared. The sample sheet and blank sheet were subjected to a tensile test under conditions of a chuck interval of 50 mm and a tensile speed of 500 mm/min using a tensile testing machine AG-X manufactured by SHIMADZU Corporation to measure a tensile strength and a tensile elongation.

### (Test results)

Tables 1 and 2 illustrate results of the above tests. Also this result indicates that by forming the cellulose nanofiber layer between the liquid impervious resin film and the exterior nonwoven fabric, wetness can be suppressed without impairing air permeability. In addition, this result also indicates that the tensile strength and the tensile elongation are improved by forming a cellulose nanofiber layer on a liquid impervious resin film formed of a moisture pervious polyethylene film.

**[Table 1]**

| | Blank diaper | Sample diaper (fiber width 300 nm) | Sample diaper (fiber width 49 nm) |
|---|---|---|---|
| Wetness (g) | 0.22 | 0.15 | 0.11 |
| Air permeability (cm³/cm²·s) | 2.2 | 2.1 | 2.3 |

**[Table 2]**

| | Blank sheet | Sample sheet |
|---|---|---|
| Tensile strength (N/mm²) | 5 | 7 |
| Tensile elongation (%) | 115 | 55 |
| Elongation at load of 9.8 N (%) | 11 | 7 |

### <Explanation of terms in specification>

The following terms in the specification have the following meanings unless otherwise specified in the specification.
- "Front-back (longitudinal) direction LD" means a direction connecting a ventral side (front side) and a dorsal side (back side), and "width direction WD" means a direction orthogonal to the front-back direction (left-right direction).
- "Unfolded state" means a flatly unfolded state without contraction or slackness.
- "Stretch rate" means a value obtained when a natural length is assumed to be 100%.
- "Gel strength" is measured as follows. To 49.0 g of artificial urine (urea: 2% by weight, sodium chloride: 0.8% by weight, calcium chloride dihydrate: 0.03% by weight, magnesium sulfate heptahydrate: 0.08% by weight, deionized water: 97.09% by weight), 1.0 g of a super absorbent polymer is added, and the resulting mixture is stirred with a stirrer. The gel thus generated is left in a thermohygrostat at 40°C × 60%RH for three hours. Thereafter, the temperature is returned to room temperature, and gel strength is measured with a curdmeter (Curdmeter-MAX ME-500 manufactured by I. Techno Engineering Co., Ltd.).
- "Basis weight" is measured as follows. A sample or a test piece is predried and then left in a test chamber or an apparatus in a standard state (test location is at a temperature of 23 ± 1°C and a relative humidity of 50 ± 2%) so as to have a constant weight. Predrying refers to causing a sample or a test piece to have a constant weight in an environment of a temperature of 100°C. Incidentally, fibers having an official moisture regain of 0.0% do not have to be predried. A sample of 100 mm × 100 mm in size is cut out from a test piece having a constant weight using a template for sampling (100 mm × 100 mm). The weight of the sample is measured. The weight is multiplied by 100 to calculate the weight per square meter to be used as a basis weight.
- "Thickness" is automatically measured under conditions that a load is 0.098 N/cm² and a pressing area is 2 cm² using an automatic thickness meter (KES-G5 handy compression tester).
- "Water absorption capacity" is measured in accordance with JIS K7223-1996 "Test method for water absorption capacity of super absorbent polymer".
- "Water absorption rate" is "time to end point" when JIS K7224-1996 "Test method for water absorption rate of super absorbent polymer" is performed using 2 g of super absorbent polymer and 50 g of physiological saline.
- In a case where environmental conditions in a test and a measurement are not described, the test and the measurement are performed in a test room or an apparatus in a standard state (test location is at a temperature of 23 ± 1°C and a relative humidity of 50 ± 2%).
- The size of each portion means a size not in a natural length state but in an unfolded state unless otherwise specified.

### Industrial Applicability

The present invention can be applied to general disposable diapers such as an underpants-type disposable diaper and a pad-type disposable diaper in addition to the tape-type disposable diaper as in the above example. Needless to say, the present invention can also be applied to another absorbent article such as a sanitary napkin.

### Reference Signs List

- 11: Liquid impervious resin film
- 12: Exterior nonwoven fabric
- 13: Connecting tape
- 13A: Connecting portion
- 13B: Tape main unit portion
- 13C: Tape attachment portion
- 15: Cellulose nanofiber layer
- 20: Target sheet
- 30: Top sheet
- 40: Intermediate sheet
- 50: Absorbent element
- 56: Absorber
- 58: Wrapping sheet
- 60: Rising gather
- 62: Gather sheet
- B: Dorsal side portion
- F: Ventral side portion
- WD: Width direction
- LD: Front-back direction

## Claims

1. An absorbent article comprising: an absorber; a liquid impervious resin film covering a back surface side of the absorber; and an exterior nonwoven fabric covering a back surface side of the liquid impervious resin film,
the liquid impervious resin film having moisture perviousness in a thickness direction, wherein
a cellulose nanofiber layer is disposed between the liquid impervious resin film and the exterior nonwoven fabric.

2. The absorbent article according to claim 1, wherein the cellulose nanofiber layer is formed on an outer surface of the liquid impervious resin film.

3. The absorbent article according to claim 2, wherein the liquid impervious resin film is a moisture pervious resin film of 10 to 25 g/m².

4. The absorbent article according to any one of claims 1 to 3, wherein the cellulose nanofiber layer is formed only in an intermediate region when the absorbent article is divided equally into three portions of a front region, the intermediate region, and a back region in a front-back direction in an unfolded state.

5. The absorbent article according to any one of claims 1 to 4, wherein the cellulose nanofiber layers are disposed at many places at intervals.

6. The absorbent article according to any one of claims 1 to 5, wherein the cellulose nanofiber layer contains cellulose nanofibers of 0.1 to 5.0 g/m².

7. The absorbent article according to any one of claims 1 to 6, wherein the exterior nonwoven fabric is a water repellent nonwoven fabric.

## Patentansprüche

1. Absorbierender Artikel, der Folgendes umfasst: ein Absorptionselement; eine flüssigkeitsundurchlässige Harzschicht, die eine Rückseite des Absorptionselements bedeckt; und einen äußeren Vliesstoff, der eine Rückseite der flüssigkeitsundurchlässigen Harzschicht bedeckt,
wobei die flüssigkeitsundurchlässige Harzschicht eine Feuchtigkeitsdurchlässigkeit in einer Dickenrichtung hat und
wobei zwischen der flüssigkeitsundurchlässigen Harzschicht und dem äußeren Vliesstoff eine Zellulose-Nanofaserlage angeordnet ist.

2. Absorbierender Artikel nach Anspruch 1, wobei die Zellulose-Nanofaserlage auf einer Außenseite der flüssigkeitsundurchlässigen Harzschicht ausgebildet ist.

3. Absorbierender Artikel nach Anspruch 2, wobei die flüssigkeitsundurchlässige Harzschicht eine feuchtigkeitsdurchlässige Harzschicht von 10 bis 25 g/m² ist.

4. Absorbierender Artikel nach einem der Ansprüche 1 bis 3, wobei die Zellulose-Nanofaserlage nur in einem Zwischenbereich ausgebildet ist, wenn der absorbierende Artikel gleichmäßig in drei Abschnitte mit einem vorderen Bereich, dem Zwischenbereich und einem hinteren Bereich in einer Richtung von vorn nach hinten in einem nicht zusammengefalteten Zustand unterteilt ist.

5. Absorbierender Artikel nach einem der Ansprüche 1 bis 4, wobei die Zellulose-Nanofaserlagen an vielen Stellen in Abständen angeordnet sind.

6. Absorbierender Artikel nach einem der Ansprüche 1 bis 5, wobei die Zellulose-Nanofaserlage Zellulose-Nanofasern von 0,1 bis 5,0 g/m² enthält.

7. Absorbierender Artikel nach einem der Ansprüche 1 bis 6, wobei der äußere Vliesstoff ein wasserabweisender Vliesstoff ist.

## Revendications

1. Article absorbant comprenant : un absorbeur ; un film de résine imperméable aux liquides recouvrant un côté de surface arrière de l'absorbeur ; et un tissu non tissé extérieur recouvrant un côté de surface arrière du film de résine imperméable aux liquides,
le film de résine imperméable aux liquides ayant une perméabilité à l'humidité dans une direction d'épaisseur, dans lequel
une couche de nanofibres de cellulose est disposée entre le film de résine imperméable aux liquides et le tissu non tissé extérieur.

2. Article absorbant selon la revendication 1, dans lequel la couche de nanofibres de cellulose est formée sur une surface externe du film de résine imperméable aux liquides.

3. Article absorbant selon la revendication 2, dans lequel le film de résine imperméable aux liquides est un film de résine perméable à l'humidité de 10 à 25 g/m².

4. Article absorbant selon l'une quelconque des revendications 1 à 3, dans lequel la couche de nanofibres de cellulose est formée uniquement dans une région intermédiaire lorsque l'article absorbant est divisé équitablement en trois parties dont une région avant, la région intermédiaire et une région arrière dans une direction avant-arrière dans un état déplié.

5. Article absorbant selon l'une quelconque des revendications 1 à 4, dans lequel les couches de nanofibres de cellulose sont disposées à de nombreux emplacements selon des intervalles.

6. Article absorbant selon l'une quelconque des revendications 1 à 5, dans lequel la couche de nanofibres de cellulose contient des nanofibres de cellulose de 0,1 à 5,0 g/m².

7. Article absorbant selon l'une quelconque des revendications 1 à 6, dans lequel le tissu non tissé extérieur est un tissu non tissé déperlant.
